# EUROPEAN PATENT SPECIFICATION

(11) **EP 0 885 191 B1**
(45) Date of publication and mention of the grant of the patent: **23.01.2002**
(21) Application number: 97906283.3
(22) Date of filing: 07.03.1997
(51) Int. Cl.: C07D 211/34

(54) **RESOLUTION OF THREO-METHYLPHENIDATE**
AUFLÖSUNGSVERFAHREN VON THREO-METHYLPHENIDATE
RESOLUTION DE THREO-METHYLPHENIDATE

(30) Priority: 08.03.1996 GB 9604943; 07.05.1996 US 16986 P
(43) Date of publication of application: 23.12.1998
(73) Proprietor: MEDEVA EUROPE LIMITED, Slough Berks SL1 3WE (GB)
(72) Inventor: ZAVAREH, Hooshang, Shahriari, Milton Road, Cambridge CB4 4WE (GB)
(74) Representative: Perry, Robert Edward
(86) International application number: GB9700643
(87) International publication number: WO9732851

(56) References cited:
- US-A- 2 957 880
- US-A- 4 196 303
- J. PHARMACOL. EXP. THER., vol. 241, no. 1, 1987, pages 152-8, XP000612231 PATRICK K. S.; CALDWELL R. W.; FERRIS R. M.; BREESE G.R.: "Pharmacology of the Enantiomers of threo-Methylphenidate" cited in the application
- PATENT ABSTRACTS OF JAPAN vol. 4, no. 73 (C-012), 28 May 1980 & JP 55 038363 A (YOSHITOMI PHARMACEUT. IND.), 17 March 1980,

## Description

### Field of the Invention

This invention relates to the resolution of *threo* methylphenidate *via* crystallisation of diastereomeric salts.

### Background of the Invention

Methylphenidate was first prepared as a mixture of the *erythro* and *threo* racemates. US-A-2957880 discloses studies upon the two racemic mixtures, which revealed that the therapeutic activity resides in the *threo* diastereomer.

The resolution of *threo* methylphenidate can be achieved using the expensive resolving agent 1,1'-binaphthyl-2,2'-diylhydrogen phosphate, a process first reported by Patrick *et al* (The Journal of Pharmacology and Experimental Therapeutics, 241:152-158 (1987)), and subsequently used by other workers in the field (e.g. Aoyama *et al*, Journal of Chromatography, 494:420 (1989)). This is perceived to be a more efficient procedure than the method disclosed in US-A-2957880, wherein the corresponding amide of *erythro* methylphenidate (i.e. R-CONR₂ rather than R-CON₂Me) is resolved with tartaric acid prior to amide hydrolysis and equilibration at the benzylic centre, followed by esterification of the resultant *threo*-acid.

An improved resolution process is described in PCT/GB97/00185. Such a resolution can be combined with the racemisation described in PCT/GB97/00281.

### Summary of the Invention

This invention is based upon the discovery that racemic *threo* methylphenidate can be resolved using inexpensive (-)-menthoxyacetic acid.

### Description of the Invention

The process of this invention may be carried out under conditions that are generally known to those skilled in the art of classical salt resolution procedures. For example, a mixture of *threo* methylphenidate free base and 1 molar equivalent of (-)-menthoxyacetic acid in an inert organic solvent is heated and then allowed to cool; the resultant precipitate is filtered, washed with an appropriate solvent and dried to afford directly a salt enriched in 98% ee *d-threo* methylphenidate. This is a great improvement on the literature method using 1,1'-binaphthyl-2,2'-diylhydrogen phosphate, described by Patrick *et al, supra*, in which the first crystallisation gave a salt corresponding to 85-90% ee material, and further recrystallisation of this material was necessary to raise the ee to 95-97%. The latter level of optical purity is achieved in the present invention in one crystallisation, with an overall higher yield. The method of this invention is therefore more efficient and more economical than the one described by Patrick *et al*.

The following Example illustrates the resolution of *threo* methylphenidate using (-)-menthoxyacetic acid.

### Example

*dl-threo* methylphenidate (1.0 g, 3.7 mmol) was suspended in water (20 ml) and treated with caustic solution. The liberated free base was extracted with MTBE (3 x 25 ml), dried over MgSO₄ and evaporated to a light oil. This was dissolved in IPA (15 ml) and heated to 60°C. (-)-Menthoxyacetic acid (0.79 g, 3.79 mmol) in IPA (5 ml) was added. Heating was continued for a further 30 min and the mixture was gradually cooled to 10°C. The resulting white crystalline product was filtered off, washed with cold IPA and dried (0.85 g, 47% by weight, corresponding to 98% ee *d-threo* methylphenidate, as determined by chiral HPLC after salt cracking).

## Claims

1. A process for resolving a mixture of enantiomers of *threo*-methylphenidate by classical salt resolution, to obtain *d-threo*-methylphenidate, **characterised in that** the resolving agent is (-)-menthoxyacetic acid.

## Patentansprüche

1. Verfahren zur Spaltung eines Gemisches von Enantiomeren von threo-Methylphenidat durch klassische Salzspaltung, um d-threo-Methylphenidat zu erhalten, **dadurch gekennzeichnet, dass** das Spaltungsmittel (-)-Menthoxyessigsäure ist.

## Revendications

1. Procédé pour dédoubler un mélange d'énantiomères de méthylphénidate thréo par dédoublement classique de sel, pour obtenir du méthylphénidate *d-thréo,* **caractérisé en ce que** l'agent de séparation est l'acide (-)-menthoxy-acétique.
